# FASCICULE DE BREVET EUROPEEN

(11) **EP 1 541 041 B1**
(45) Date de publication et mention de la délivrance du brevet: **19.10.2011**
(21) Numéro de dépôt: 04292941.4
(22) Date de dépôt: 10.12.2004
(51) Int. Cl.: A23L 1/305, A23L 1/302, A61K 31/661, A61K 31/513, A61K 31/4015, A61K 31/355, A61K 31/201, A61K 31/198, A61K 31/185, A61K 45/06, A61P 25/00

(54) **Compositions diététiques contre le stress**
Diätetische Zusammensetzungen gegen Stress
Dietary compositions against stress

(30) Priorité: 10.12.2003 FR 0314451
(43) Date de publication de la demande: 15.06.2005
(73) Titulaire: Synergia Holding, 1860 Aigle (CH)
(72) Inventeur: Predal Ludovic, 42015 Saint-Etienne Cedex 2 (FR)
(74) Mandataire: Tezier Herman, Béatrice

(56) Documents cités:
- WO-A-00/30477
- WO-A-91/11117
- WO-A-02/076436
- WO-A-03/013495
- DE-U- 29 709 820
- FR-A- 2 704 391
- FR-A- 2 704 392
- US-B1- 6 391 332
- DATABASE BIAM 05 Avril 2001 'Lithium Carbonate'
- 'Fiche VIDAL du produit D-Stress', [en ligne] Extrait de l'Internet: <URL:http://www.vidal.fr/>
- SUENAGA R ET AL: "Intracerebroventricular injection of L-arginine induces sedative and hypnotic effects under an acute stress in neonatal chicks", AMINO ACIDS (VIENNA), vol. 35, no. 1, June 2008 (2008-06), pages 139-146, ISSN: 0939-4451
- DATABASE MEDLINE [en ligne] US NATIONAL LIBRARY OF MEDICINE (NLM), BETHESDA, MD, US 01 Janvier 2001 SUFKA K J ET AL: 'Anxiolytic properties of botanical extracts in the chick social separation-stress procedure.' Database accession no. NLM11205422 & PSYCHOPHARMACOLOGY 1 JAN 2001 LNKD- PUBMED:11205422, vol. 153, no. 2, 1 January 2001 (2001-01-01), pages 219-224, ISSN: 0033-3158
- DATABASE MEDLINE [en ligne] US NATIONAL LIBRARY OF MEDICINE (NLM), BETHESDA, MD, US Mars 2009 SUFKA KENNETH J ET AL: 'Antidepressant efficacy screening of novel targets in the chick anxiety-depression model.' Database accession no. NLM19300238 & BEHAVIOURAL PHARMACOLOGY MAR 2009 LNKD- PUBMED:19300238, vol. 20, no. 2, March 2009 (2009-03), pages 146-154, ISSN: 1473-5849
- DATABASE MEDLINE [en ligne] US NATIONAL LIBRARY OF MEDICINE (NLM), BETHESDA, MD, US Février 2004 FELTENSTEIN MATT W ET AL: 'The chick separation stress paradigm: a validation study.' Database accession no. NLM14751448 & PHARMACOLOGY, BIOCHEMISTRY, AND BEHAVIOR FEB 2004 LNKD- PUBMED:14751448, vol. 77, no. 2, February 2004 (2004-02), pages 221-226, ISSN: 0091-3057

## Description

La présente invention se rapporte au domaine de la chimie.

Elle a plus spécifiquement pour objet de nouvelles formulations destinées à l'alimentation, à la diététique et aux compléments alimentaires destinés à compenser les effets du stress.

Afin d'illustrer l'art antérieur, la demande de brevet français FR 2 704 391. concerne un complément nutritionnel pour faciliter l'adaptation au stress quotidien. Le modèle d'utilité allemand DE 297 09 820 concerne un additif spécial pour produit alimentaire. Le brevet américain US 6 391 332 concerne des compositions de micronutriments pour le traitement des traumatismes, brûlures et maladies graves.

L'invention est définie par les revendications.

Elle a plus précisément pour objet une utilisation non-thérapeutique de nouvelles compositions diététiques pour combattre les effets du stress chez l'adulte, **caractérisées en ce qu'**elles renferment un sel de magnésium avec un acide organique, liposoluble ; des aminoacides choisis parmi la Taurine et l'Arginine et une ou des vitamines du groupe B, associées ou en mélange avec un excipient ou un véhicule inerte, non toxique, pharmaceutiquement-acceptable.

Le terme sel ou sels de magnésium avec un acide organique, liposolubles se rapporte à l'utilisation comme ingrédient actif d'un sel de magnésium d'un acide aliphatique ou cyclanique comme par exemple le pidolate de magnésium (pyrrolidone 2-carboxylate), l'orotate de magnésium (acide 1,2 3,6 tétrahydro 2,6 dioxo 4-pyrimidine 6-carboxylique), le cyclohexyl acétate de magnésium, le cyclopentyl propionate de magnésium ou l'oléate de magnésium.

De la même façon on peut utiliser un glycérophosphate de magnésium, un glucose 6-phosphate de magnésium, un arabinose phosphate de magnésium ou le glucose 2,6 diphosphate de magnésium.

Le mélange contient également de l'Arginine et de la Taurine. Les aminoacides peuvent être contenus dans la formulation sous forme racémique ou optiquement active, sous forme libre ou salifiée par un acide minéral ou organique.

Parmi les sels de Taurine ou d'Arginine on citera plus particulièrement :
- les chlorhydrates,
- les sulfates,
- les phosphates,
- les méthanes sulfonates,
- les acétates,
- les gluconates,
- les saccharates,
- les lactates,
- les iséthionates,
- les nicotinates,
- les pidolates ou
- les glycérophosphates

La Taurine peut également être utilisée sous forme de sel avec une base minérale.

Les vitamines du groupe B englobent essentiellement la vitamine B1 et ses dérivés, la vitamine B2, la vitamine B5, les vitamines PP, la vitamine B6, la vitamine B8, la vitamine B9, l'acide pantothénique, les vitamines B12. On y rattache également les vitamines du groupe des tocophérols.

Ces vitamines peuvent être utilisées sous forme de produits purs trouvés dans le commerce. Plus souvent on préfère ne pas utiliser des composés de synthèse mais utiliser les vitamines sous formes de totum vitaminique tel qu'on peut l'isoler de levure de bière ou d'extraits de foie.

Les tocophérols peuvent être de l'α-tocophérol, du β-tocophérol, du δ-tocophérol, de l'ε-tocophérol ainsi que des tocotriénols α, β ou γ.

Les tocophérols et/ou les tocotriénols peuvent exister sous forme phénolique libre ou estérifiée comme par exemple un acétate ou un succinate acide.

Les compositions employées selon l'invention peuvent être préparées par incorporation successive des différents constituants, en particulier du ou des sels de magnésium, des aminoacides puis des vitamines à un excipient ou un véhicule inerte approprié pour la voie digestive.

L'excipient inerte peut être un agent diluant solide comme le carbonate de calcium, le phosphate tricalcique, le lactose, la cellulose micro-cristalline, l'éthylcellulose, l'hydroxypropylcellulose, l'hydroxypropylméthyl cellulose, la silice colloïdale, le sulfate de calcium le silicate de magnésium, le trisilicate de magnésium, les argiles, les bentonites, les zéolithes, le carboxyméthyl amidon, la polyvinylpyrrolidone réticulée ou encore la carboxyméthylcellulose.

Le véhicule inerte peut être un véhicule huileux ou un véhicule glycérique susceptible de disperser ou même de mettre en solution une partie ou la totalité des ingrédients actifs.

Un véhicule particulièrement approprié est un mélange renfermant essentiellement de l'huile d'olives et de la cire d'abeilles. La cire d'abeilles est principalement de la cire d'abeilles jaune. Le mélange d'huile d'olives et de cire d'abeilles peut être additionné de doses élevées de vitamine C qui joue un rôle d'antioxydant ainsi que de bêta carotène, de lutéine, de naringine, d'hespéridine ou de gallate d'isopropyle. Ces ingrédients supplémentaires sont destinés à être additionnés aux compositions selon l'invention.

Les compositions diététiques employées selon l'invention ainsi réalisées sont destinées à combattre les effets du stress chez l'adulte. Elles évitent ainsi les phénomènes d'hyper émotivité fréquents chez les sujets stressés.

Les compositions employées selon l'invention sont conditionnées sous une des formes qui rendent possible l'administration par voie digestive notamment sous forme de capsules, de gélules, de comprimés, de granulés, de pilules, de cachets, de poudres aromatisées ou non, de dragées, de comprimés enrobés ou pelliculés, sécables ou non sécables, de comprimés à mâcher, à sucer ou de comprimés adhérents à la cavité buccale.

Les compositions employées selon l'invention peuvent également se présenter sous forme liquide, pâteuse ou visqueuse comme par exemple des gels, des laits, des sirops, des émulsions huile dans l'eau ou eau dans l'huile, des solutions dans des mélanges de triglycérides comme le produit commercialisé sous la dénomination INTRALIPID.

Les compositions employées selon l'invention sont destinées à être administrées de 1 à 4 fois par jour, de préférence au moment des repas.

La teneur en sel de magnésium s'échelonne de 100 à 300 mg par prise unitaire. La teneur en Arginine exprimée en L-Arginine s'échelonne de 10 mg à 120 mg par prise unitaire. La teneur en Taurine s'échelonne de 10 mg à 80 mg par prise unitaire.

Les exemples suivants illustrent l'invention. Ils ne la limitent en aucune façon.

### EXEMPLE I

| | |
|---|---|
| Glycérophosphate de magnésium | 200 g |
| Mélange d'aminoacides | 150,97 g |
| Trisilicate de magnésium | 35 g |
| Carbonate de magnésium | 30 g |
| Stéarate de magnésium végétal | 7 g |
| Vitamine PP | 2 g |
| Vitamine B1 | 0,2 g |
| Vitamine B5 | 0,2 g |
| Vitamine B6 | 0,2 g |
| Vitamine B2 | 0,1g |
| Vitamine B8 | 0,01 g |
| Vitamine B9 | 0,02 g |
| Excipient pour 1.000 comprimés terminés à 450 mg | |

### Composition du mélange d'aminoacides

| | |
|---|---|
| L-Arginine HCL | 100 mg |
| Taurine | 35 mg |
| Carbonate de Calcium | 5 mg |
| Poudre d'acides gras non saturés | 5 mg |
| Vitamine PP | 3mg |
| Vitamine B1 | 0,8 mg |
| Vitamine B5 | 0,8 mg |
| Vitamine B6 | 0,8 mg |
| Vitamine B2 | 0,4 mg |
| Vitamine B8 | 0,09 mg |
| Vitamine B9 | 0,08 mg |
| Formule anti-oxydante | 0,05 mg |

**Formule anti-oxydante**

| | |
|---|---|
| Mélange d'huile d'olives et de cire d'abeilles | 455,2 mg |
| Vitamine C | 120 mg |
| Vitamine E naturelle | 10 mg |
| Bêta carotène | 4,8 mg |
| Lycopène | 1 mg |
| Lutéine | 1 mg |

### EXEMPTE II

### Composition à base de pidolate de magnésium

| | |
|---|---|
| Pidolate de magnésium | 180 g |
| L-Arginine chlorhydrate | 40 g |
| Thiamine chlorhydrate | 0,2 g |
| Pyridoxine | 0,2 g |
| Taurine | 60 g |
| Vitamine PP | 1 g |
| Vitamine B2 | 0,1 g |
| Acide pantothénique | 0,4 g |
| Excipient qsp 1.000 comprimés terminés à 0,350 g | |

## Revendications

1. Utilisation non-thérapeutique de nouvelles compositions diététiques pour combattre les effets du stress chez l'adulte, **caractérisée en ce que** lesdites compositions renferment un ou plusieurs sels de magnésium avec un acide organique, liposolubles, de la Taurine, de l'Arginine et une ou plusieurs vitamines du groupe B, associées ou en mélange avec un excipient ou un véhicule inerte non-toxique pharmaceutiquement-acceptable.

2. Utilisation selon la revendication 1, **caractérisée en ce que** lesdites compositions sont sous forme de capsules, gélules, comprimés ou granulés, conditionnés sous une forme qui rend possible l'administration par voie digestive.

3. Utilisation selon la revendication 1 ou 2, dans laquelle le sel de magnésium liposoluble est le glycérophosphate de magnésium.

4. Utilisation selon la revendication 1 ou 2, dans laquelle le sel de magnésium liposoluble est le pidolate de magnésium.

5. Utilisation selon la revendication 1 ou 2, dans laquelle le sel de magnésium liposoluble est l'orotate de magnésium.

6. Utilisation selon la revendication 1 ou 2, dans laquelle le sel de magnésium liposoluble est l'oléate de magnésium.

7. Utilisation selon la revendication 1 ou 2, dans laquelle l'Arginine est présente sous forme de sel avec un acide minéral ou organique.

8. Utilisation selon la revendication 1 ou 2, dans laquelle la Taurine est présente sous forme de sel avec une base minérale.

9. Utilisation selon la revendication 1 ou 2, dans laquelle les vitamines du groupe B englobent la vitamine B1, la vitamine B2, la vitamine B5, la vitamine B6, la vitamine B8, la vitamine B9, l'acide pantothénique et les vitamines B12.

10. Utilisation selon la revendication 9, dans laquelle des vitamines du groupe E sont également présentes.

11. Utilisation selon la revendication 1 ou 2, dans laquelle l'excipient est un agent diluant solide approprié pour la voie digestive.

12. Utilisation selon la revendication 1 ou 2, dans laquelle le véhicule inerte est un véhicule huileux ou un véhicule glycérique approprié pour la voie digestive.

13. Utilisation selon la revendication 1 ou 2, dans laquelle le véhicule renferme essentiellement de l'huile d'olive et de la cire d'abeille.

14. Utilisation selon la revendication 13, dans laquelle lesdites compositions comprennent en outre des doses élevées de vitamine C, du bêta-carotène, de la lutéine, de la naringine, de l'hespéridine ou du gallate d'isopropyle.

## Claims

1. Non-therapeutic use of new dietetic compositions to combat stress effects in adult subject, **characterized in that** said compositions comprise one or several liposoluble magnesium salts with an organic acid, taurine, arginine and one or several vitamins of group B, associated or in mixture with a non toxic pharmaceutically acceptable excipient or inert carrier.

2. Use according to claim 1, **characterized in that** said compositions are in the form of capsule, hard gelatin capsule, tablet or granule, packaged in a form suitable for administration through the digestive tract.

3. Use according to claim 1 or 2, wherein the liposoluble magnesium salt is magnesium glycerophosphate.

4. Use according to claim 1 or 2, wherein the liposoluble magnesium salt is magnesium pidolate.

5. Use according to claim 1 or 2, wherein the liposoluble magnesium salt is magnesium orotate.

6. Use according to claim 1 or 2, wherein the liposoluble magnesium salt is magnesium oleate.

7. Use according to claim 1 or 2, wherein arginine is present in the form of a salt with mineral or organic acid.

8. Use according to claim 1 or 2, wherein taurine is present in the form of a salt with mineral base.

9. Use according to claim 1 or 2, wherein group B vitamins encompass vitamin B1, vitamin B2, vitamin B5, vitamin B6, vitamin B8, vitamin B9, pantothenic acid and vitamins B12.

10. Use according to claim 9, wherein vitamins of group E are also present.

11. Use according to claim 1 or 2, wherein the excipient is a solid diluting agent suitable for digestive tract.

12. Use according to claim 1 or 2, wherein the inert carrier is oil carrier or glyceric carrier suitable for digestive tract.

13. Use according to claim 1 or 2, wherein the carrier comprises essentially olive oil and beeswax.

14. Use according to claim 13, wherein said compositions further comprise high doses of vitamin C, beta-carotene, lutein, naringin, hesperidin or isopropyl gallate.

## Patentansprüche

1. Nicht-therapeutische Verwendung neuartiger diätetischer Zusammensetzungen zur Bekämpfung von Stresseffekten beim Erwachsenen, **dadurch gekennzeichnet, dass** besagte Zusammensetzungen ein oder mehrere fettlösliche Magnesiumsalze einer organischen Säure, Taurin, Arginin und ein oder mehrere Vitamine der B-Gruppe enthalten, die mit einem nichttoxischen, inerten, pharmazeutisch verträglichen Arzneimittelträger oder Vehiculum assoziiert oder vermischt sind.

2. Verwendung gemäß Anspruch 1, **dadurch gekennzeichnet, dass** besagte Zusammensetzungen als Kapseln, Geletinekapseln, Tabletten oder Granulate vorliegen und in einer Form präpariert sind, welche die Verabreichung über den Verdauungsweg ermöglicht.

3. Verwendung gemäß Anspruch 1 oder 2, wobei das fettlösliche Magnesiumsalz Magnesiumglycerinphosphat ist.

4. Verwendung gemäß Anspruch 1 oder 2, wobei das fettlösliche Magnesiumsalz Magnesiumpidolat ist.

5. Verwendung gemäß Anspruch 1 oder 2, wobei das fettlösliche Magnesiumsalz Magnesiumorotat ist.

6. Verwendung gemäß Anspruch 1 oder 2, wobei das fettlösliche Magnesiumsalz Magnesiumoleat ist.

7. Verwendung gemäß Anspruch 1 oder 2, wobei Arginin als Salz einer organischen oder anorganischen Säure vorliegt.

8. Verwendung gemäß Anspruch 1 oder 2, wobei Taurin als Salz einer anorganischen Base vorliegt.

9. Verwendung gemäß Anspruch 1 oder 2, wobei die Vitamine der B-Gruppe Vitamin B1, Vitamin B2, Vitamin B5, Vitamin B6, Vitamin B8, Vitamin B9, Pantothensäure und die Vitamine B12 umfassen.

10. Verwendung gemäß Anspruch 9, wobei Vitamine der E-Gruppe ebenfalls vorliegen.

11. Verwendung gemäß Anspruch 1 oder 2, wobei der Arzneimittelträger ein festes Verdünnungsmittel ist, das für den Verdauungsweg geeignet ist.

12. Verwendung gemäß Anspruch 1 oder 2, wobei das inerte Vehiculum ein Vehiculum auf ÖlBasis oder ein Vehiculum auf Glycerin-Basis ist, das für den Verdauungsweg geeignet ist.

13. Verwendung gemäß Anspruch 1 oder 2, wobei das Vehiculum im Wesentlichen Olivenöl oder Bienenwachs umfaßt.

14. Verwendung gemäß Anspruch 13, wobei besagte Zusammensetzungen außerdem erhöhte Dosen an Vitamin C, beta-Carotin, Lutein, Naringin, Hesperidin oder Isopropylgallat umfassen.
